# EUROPEAN PATENT SPECIFICATION

(11) **EP 4 257 578 B1**
(45) Date of publication and mention of the grant of the patent: **03.07.2024**
(21) Application number: 23166812.0
(22) Date of filing: 05.04.2023
(51) Int. Cl.: C07C 37/82, C07C 37/84, C07C 39/23, C07C 51/47, C07C 65/19, A61K 36/00, A61K 31/352, C07C 37/00, A61K 36/185

(54) **PROCESS FOR THE EXTRACTION OF HIGH PURITY CANNABINOIDS**
VERFAHREN ZUR EXTRAKTION VON HOCHREINEN CANNABINOIDEN
PROCÉDÉ D'EXTRACTION DE CANNABINOÏDES DE GRANDE PURETÉ

(30) Priority: 06.04.2022 IT 202200006770
(43) Date of publication of application: 11.10.2023
(73) Proprietor: S.A.L.A.R.S. Società Azionaria Laboratori Alcaloidi Rifornimenti Sanitari S.p.A., 22100 Como (IT)
(72) Inventor: CASTELLI, Curzio Vittorio, 20123 Milano (IT); CARAFFI, Claudio, 22042 Como (IT); SPIGA, Marco, 09012 Cagliari (IT); LA LOGGIA, Claudia, 22042 Como (IT)
(74) Representative: Santoro, Sofia

(56) References cited:
- WO-A1-2019/057994
- US-A1- 2018 280 459
- US-B2- 10 301 242

## Description

### Field of the invention

The present invention relates to the field of extraction of active ingredients from plants or plant-derived material and, in particular, the invention relates to a process for extracting wax-free (-)-cannabidiol (CBD) from hemp or cannabis, particularly *Cannabis sativa L.,* which can be used either as an oil or as a starting material to obtain substantially pure CBD for pharmaceutical and medical uses.

### Background of the invention

Cannabis or rather *Cannabis sativa L.* has been used in medicine for centuries. The medical benefits of cannabis extracts were first discovered in 2900 b.C. in China, and by 1522 a.C. they were described in the *"Herbarium Vulgare"* of the Camaldolesi Monks as a remedy to relieve certain types of pain. In Victorian times then cannabis extracts were used as a prescription medicine, Queen Victoria herself used it against menstrual pain, and more generally it was used as a sedative to treat "hysteria, delirium, epilepsy, nervous insomnia, migraine, pain and dysmenorrhea." Historically, cannabis was also considered by many physicians to be the only treatment capable of combating pain resistant to opioid analgesics, useful in treating conditions such as spinal cord injury and other forms of neuropathic pain, including pain and spasm in multiple sclerosis.

Cannabis use continued in the terms described above until the mid-twentieth century, when cannabis began to be used for recreational purposes. At that point, more restrictive legislation led to the prohibition of its use. However, the usefulness of cannabis as a prescription medicine has been reevaluated today, and the discovery of specific cannabinoid receptors and new methods of administration have enabled the expanded use of cannabis-based medicines with more targeted and novel indications.

The term cannabinoids generally identifies a family of chemical compounds found in *Cannabis sativa.* Cannabinoids or cannabidiols are chemical substances of natural origin and biochemically classified as terpenophenols. They are compounds that share the ability to interact with cannabinoid receptors.

About seventy such compounds have been identified to date. The main cannabinoid constituents identified as present in cannabis are tetrahydrocannabidiolic acid (THCA) and cannabidiolic acid (CBDA), precursors of the neutral cannabinoids, tetrahydrocannabinol (THC) and cannabidiol (CBD), respectively, which are not present on the fresh plant.

CBD was previously considered an inactive constituent. Today, numerous clinical studies demonstrate its pharmacological activity, although larger studies on its pharmacology remain necessary to fully explore its pharmaceutical potential; therefore, there is a need for substantially pure CBD preparations for use in such studies.

Drugs such as Sativex, a drug extracted from *Cannabis Sativa,* with standardized cannabinoid (THC and CBD) content, and Epidiolex, a drug containing pure CBD in oil, obtained from *Cannabis Sativa* extracts, are a first step in the continuing search for the importance of these molecules as active ingredients.

Cannabinoids are found in the hemp plant, or *Cannabis sativa,* in the form of their carboxylic derivatives, the cannabinoid carboxylic acids THCA and CBDA, from which the so-called "neutral cannabinoids" are derived by decarboxylation, that is, by CO₂ removal. Thus, for example, cannabidiol (CBD) is formed by decarboxylation of cannabidiolic acid (CBDA).

However, extraction of pure products from hemp is made difficult by the fact that waxes are present in the hemp plant, both on the leaves and inflorescences, which must be removed to recover pure CBD and THC extracts. Waxes are a class of aliphatic compounds that include hydrocarbons, polycosanols, fatty acids, fatty aldehydes, *etc*.; it is known to be necessary to remove this material before further processing as it creates problems in the thermodynamics of separation. The process of removing these waxes generally takes place after solvent extraction (either conventional solvents or supercritical CO₂ used as a solvent), either by the so-called "winterization" process or by dual-phase separation in which the waxes are confined in the most affine solvent that does not contain the cannabinoids.

In addition, purification of the extracted products can also be done by extraction with oils (such as olive oil, hemp oil, sesame oil, etc.), which normally do not extract the waxes, however, and remain as oils in the extracted products.

In light of the renewed interest in cannabis derivatives such as THC and CBD in the medical and pharmaceutical fields, the technical problem of having a process for the extraction from plant material of their precursors THCA and CBDA, free of waxes and solvents, and usable, without any purification, as such or as starting products to obtain, after decarboxylation, THC, CBD or other neutral cannabinoids in crystalline form of high purity, is therefore very much in felt.

### Summary of the invention

The present invention solves the above problems by a process for producing wax-free extracts directly during the extraction step of fresh, dried or frozen cannabis plant biomass to obtain purified extracts used as such or to obtain CBD or other neutral cannabinoid, leaving the waxes in the organic waste material. The present process also solves the industrial problem related to the use of solvents with high flammability or used at high pressures, during the extraction step in known processes, with safety-related risks.

Surprisingly, it has been seen that the extraction process described in the present invention can be applied to "biomass" having any ratio between the acid (CBDA) and decarboxylated (CBD) forms through a single process that leaves the waxes in the extracted biomass while avoiding their removal later after extraction.

This innovative system eliminates the winterization step or other subsequent wax removal techniques. It is also ameliorative compared to extraction using oils (olive, hemp, etc.) in which normally the waxes are not extracted but then it becomes difficult to remove the oil to make a concentrated solvent-free extract that could be used as such or used for crystallization or purification of cannabinoids.

It is therefore an object of this invention a process for producing an extract from a cannabis plant biomass high in cannabidiolic acid (CBDA) and/or cannabidiol (CBD) or other cannabinoid, free of waxes and extraction solvents, the process being as defined in claim 1 appended hereto.

Further important features of the preparation process of this invention are defined in the depended claims appended hereto.

### Brief description of the Drawings

- Figure 1:: CBD pure crystal obtained from fresh hemp (Example 2).
- Figure 2:: CBD pure crystal obtained from dried hemp with a 60/40 CBDA/CBD ratio (Example 3).
- Figure 3:: purified wax-free extract with CBD and CBDA not fully decarboxylated (Example 4).
- Figure 4:: purified and decarboxylated wax-free extract obtained from dried hemp with an 85/15 CBDA/CBD ratio (Example 5).
- Figure 5:: Pure crystal CBD obtained from dried hemp with an 85/15 CBDA/CBD ratio (Example 5).
- Figure 6:: purified wax-free extract from 2 combined extractions of hemp with high CBDA content (Example 6).
- Figure 7:: CBD purified wax-free extract from 2 combined extractions of hemp with high CBDA content (Example 6).
- Figure 8:: purified wax-free extract obtained from dried hemp extracted with water and acetone (Example 7).
- Figure 9:: CBD pure crystal obtained from dried hemp extracted with water and acetone (Example 7).
- Figure 10:: crude extract decarboxylated at 80°C after step ii (Example 10).
- Figure 11:: ¹H NMR spectrum in CD₃OD of the crude wax-free concentrated extract (Example 1).
- Figure 12:: ¹H NMR spectrum in CD₃OD of the concentrated extract obtained from the extraction of spent hemp with the presence of waxes (Example 9).
- Figure 13:: ¹H NMR spectrum in CD₃OD of pure crystal CBD (Example 6 Figure 7).
- Figure 14:: ¹H NMR spectrum in CD₃OD of the concentrated extract obtained from the extraction of spent hemp with the presence of waxes (Example 13).
- Figure15:: CBGA pure crystal obtained from a wax-free high CBG/CBGA hemp extract (Example 12).
- Figure 16:: ¹H NMR spectrum in CDCl₃ CBGA pure crystal obtained from a high CBG/CBGA wax-free hemp extract (Example 12).
- Figure17:: CBDA salt of dimethylcyclohexylammonium (DMCEA) pure crystal from a wax-free high CBDA hemp extract (Example 14).
- Figure 18:: CBD pure crystal obtained by decarboxylation of CBDA salt of dimethylcyclohexylammonium (DMCEA) pure crystal (Example 15).
- Figure 19:: CBG pure crystal obtained by decarboxylation of pure crystal CBGA (Example 16).
- Figure 20:: ¹H NMR spectrum in CDCl₃ CBG pure crystal obtained by decarboxylation of CBGA pure crystal (Example 16).

### Detailed description of the invention

The main objective of the present invention is to provide a process for the preparation of cannabis extracts that is easily implementable on an industrial scale, reproducible regardless of the initial cannabinoid content, and above all that allows obtaining an extract already free of waxes, or more generally free from process by-products (e.g., extraction solvents), without necessarily having to resort to expensive purification procedures such as, for example, chromatography purification.

The process of this invention comprises an initial step of contacting a cannabis plant biomass containing CBDA if fresh, or CBD and/or CBDA if dried, or other cannabinoid either neutral or in carboxylic acid form, with a mixture of water and water-miscible solvent, preferably a hydroalcoholic mixture, to give a suspension, in which the suspension is kept under agitation for a time of at least 10 minutes, preferably at least 30 minutes, without adjusting the pH.

After extensive experimentation, the inventors of the present invention have found that by macerating a cannabis plant biomass with a mixture of water and a water-miscible solvent, wherein water and water-miscible solvent are present in a weight ratio of between 3:1 and 1:1, more preferably 1:1, yields a wax-free cannabis extract.
ii) correct the pH of the suspension obtained from step i) to pH between 6.5 and 10.5, if necessary, and filter away the biomass; iii) extract the filtrate by addition of an organic solvent immiscible with said mixture, acidify to pH between 5.5 and 4.0, and recover the organic phase.

A person skilled in the field will understand that when using fresh or frozen cannabis (which unlike dried cannabis contains higher or lower amounts of water) said weight ratio of water to water-miscible solvent is met by taking into consideration the amount of water contained in the starting plant biomass. In other words, the weight ratio between 3:1 and 1:3 is to be understood as the ratio of total water (i.e., water possibly present in the starting plant biomass + water added as an extraction solvent) to solvent. The water possibly present in the starting plant biomass can be measured as known in the art, for example, by the Loss-on-Drying (LOD) method and/or the Karl Fischer (KF) method.

Therefore, the present invention relates to a process for producing a wax-free cannabis extract, said process comprising the step of:
i) suspending a cannabis plant biomass with a mixture of water and water-miscible solvent, such that the weight ratio of total water to water-miscible solvent in the suspension is between 3:1 and 1:1, and maintaining the suspension under stirring for a time of at least 10 minutes, preferably at a temperature not exceeding 50°C, without adjusting the pH.
ii) correct the pH of the suspension obtained from step i) to pH between 6.5 and 10.5, if necessary, and filter away the biomass; iii) extract the filtrate by addition of an organic solvent immiscible with said mixture, acidify to pH between 5.5 and 4.0, and recover the organic phase.

For the purposes of the present invention, water-miscible solvents chosen from alcohols, acetone, acetonitrile or mixtures thereof are particularly suitable. Alcohols herein preferably mean C1-C4 alcohols, such as methanol, ethanol, n-propanol, and i-propanol, preferably ethanol or methanol. In particular, the water-miscible solvents of the present invention are chosen from ethanol, methanol, acetone, acetonitrile, or mixtures thereof.

The process of the invention can be conducted over a wide range of temperatures, however, as specified above, the process is preferably conducted at temperatures not exceeding 50°C to minimize the risk of obtaining an extract that contains an amount, albeit minimal, of waxes. Advantageous results, especially in terms of yield, occur when the suspension is maintained at a temperature between 0°C and 50°C, preferably between 5°C and 45°C, more preferably between 10°C and 25°C or 15°C and 25°C. By increasing the amount of water and decreasing the amount of solvent from the indicated 3:1 weight ratio, it is possible to conduct the process and obtain a wax-free extract even at temperatures higher than 50°C. However, high process temperatures are disadvantageous from an industrial point of view. For the same reasons, by decreasing the amount of water and increasing the amount of solvent from the indicated 3:1 weight ratio, it is possible to conduct the process and obtain a wax-free extract even at temperatures below 0°C.

The suspension is kept under agitation in the mixture of water and water-miscible solvent for a sufficient time to extract the bioactive compounds from the hemp biomass, for example, for a time of at least 10 minutes, preferably for a time of at least 30 minutes.

In this first stage, the pH is not corrected, specifically no alkalizing agents are added such as, just to name a few, sodium hydroxide, potassium hydroxide, potassium carbonate, sodium carbonate, *etc.* In fact, without wishing to be bound by any theory, the inventors noted that when step i) of extraction in hydroalcoholic mixture is carried out in the presence of alkalizing agents, or otherwise at a pH greater than 10.5, an extract of lower purity is obtained (Comparative Example 11b). Filtering the suspension obtained from step i) yields a wax-free cannabis extract (see Example 1). Therefore, the process according to any of the embodiments described here can further include step ii) of filtering away the biomass.

The inventors observed that when the pH of the suspension obtained from step i) is between 6.5 and 10.5, extremes included, the wax-free cannabis extract is obtained in greater quantities and therefore with higher yields. Moreover, the pH correction allows the process of the invention to be employed regardless of the cannabinoid content of the starting plant biomass, particularly regardless of its CBD / CBDA content, as will be evident from the examples that follow. For these reasons, if the pH value of the suspension obtained from step i) is not between 6.5 to 10.5, it is preferable to correct/adjust the pH before filtration.

The process according to any of the forms of implementation described herein further includes step ii) of correcting the pH of the suspension obtained in step i) to pH between 6.5 and 10.5, if necessary, and filtering away the biomass.

The pH can be corrected according to any of the methods known in the art, for example, by adding soda, potash, a mixture thereof, or even a buffer solution in a suitable amount to bring the pH into the indicated range.

By way of example, but in no way limiting, step ii) of pH correction may be accomplished by the addition, under agitation, of an alkaline solution, preferably a 30 percent sodium hydroxide (NaOH) solution, bringing the pH between 6.5 and 10.5, more preferably between 7.0 and 8.0, or a basic buffer. Adjusting the pH to these values is also particularly advantageous because it allows the elimination of fatty acids such as omega-3 or omega-6 that may sometimes be present in the starting biomass.

After removal of the biomass, the filtered solution of water and water-miscible solvent (or more simply the filtrate), specifically including acidic or salified CBDA and/or CBD, is recovered according to any of the known methods of the art.

The filtrate obtained, *i.e.,* the wax-free cannabis extract, can be used as such or be suitably processed to obtain an extract containing neutral CBD and/or CBG, CBDA and/or CBGA, or a mixture of the two.

Should the choice be made to further process the filtrate, it can be used as such in subsequent steps iii) or iv) or it can be appropriately concentrated to reduce volumes prior to further processing.

The filtrate obtained from step ii) can either be directly subjected to decarboxylation by heating the suspension to a temperature between the boiling temperature of the solvent and 180°C (Example 10) or the filtrate can be added with an organic solvent immiscible with water, such as heptane or a mixture of its isomers, hexane or a mixture of its isomers, and acidified to a pH between 4.0 and 6.0, preferably between 4.5 and 5.5 (Examples 2, 3, 5, 6, 7, 8 and 9). The organic phase is then recovered and, if desired, then subjected to decarboxylation as mentioned above.

As will be evident from the following examples, pH correction is carried out on the hydroalcoholic phase and then alternately directly on the filtrate obtained from step ii) or after addition with the organic solvent. Moving the pH to acidic values favors the extraction of cannabinoids - especially those in acidic form - in the heptane or hexane phase.

Therefore, the process according to any of the embodiments of the invention comprises an extraction step iii) on the filtrate by addition of an organic solvent immiscible with the extraction mixture employed in step i), acidification to a pH between 5.5 and 4.0, and recovery of the organic phase.

Said organic solvent immiscible with water is preferably a C5-C8 hydrocarbon, more preferably n-heptane or a mixture of its isomers or hexane or a mixture of its isomers.

This two-phase separation with apolar solvent is useful to purify the filtrate obtained from step ii).

The organic phase obtained from step iii) once concentrated will be solvent-free and can be used as such or decarboxylated as better described below (Example 4).

Therefore, according to a preferred embodiment, the present invention relates to a process for producing a wax-free cannabis extract, said process comprising the following steps:
**i)** suspend a cannabis plant biomass with a mixture of water and water-miscible solvent such that in the suspension the weight ratio of total water to water-miscible solvent is between 3:1 and 1:1 and keep the suspension under stirring for at least 10 minutes, at a temperature not exceeding 50°C, without adjusting the pH;
**ii)** correct the pH of the suspension obtained from step i) to pH between 6.5 and 10.5, if necessary, and filter away the biomass;
**iii)** extract the filtrate by addition of an organic solvent immiscible with said mixture (i.e., the mixture of water and solvent miscible with water used in step i), acidify to pH between 5.5 and 4.0, and recover the organic phase.

Thereafter, the filtrate obtained from step ii) or the organic phase obtained from step iii) may be subjected to step iv) of decarboxylation by any of the known techniques of the art, such as by heating the material to a temperature between the boiling temperature of the solvent and 180°C.

Thus, the process according to any of the embodiments of the invention may optionally also comprise a step iv) of decarboxylating the filtrate recovered from step ii) or the organic phase recovered from step iii) by heating the material to a temperature between that of boiling of the solvent and 180°C.

By way of example, but in no way limiting, if the solvent used is methanol/ethanol, decarboxylation will take place at a temperature between 65 and 80°C to reflux for between 24 and 72 hours; if decarboxylation takes place directly in the solvent-free oil, decarboxylation will take place at a temperature between 100 and at 140°C in between 1 and 12 hours.

This yields an extract containing cannabidiols that can be used as such or processed further and/or used, for example, to crystallize CBD or CBG.

As an example, the extract can optionally be subjected to filtration on silica to remove any by-products or polar residues akin to silica, in particular by diluting in 2-6 volumes of appropriate organic solvent, immiscible with the extraction mixture used in step i), e.g., in heptane, the product obtained from the previous decarboxylation step iv) and filtering indeed on silica, optionally moistened with the selected organic solvent.

Crystallization of cannabinoids, particularly CBD and/or CBG, preferably occurs in heptane or hexane, more preferably heptane mixture of isomers.

Therefore, the process according to any of the described embodiments may further comprise a step v) of crystallization of cannabinoids (CBD and/or CBG) from heptane or hexane that may succeed step iv) of decarboxylation, optionally also downstream of the filtration step on silica.

The decarboxylation step iv) is necessary when an extract including CBG or CBD free of waxes and reaction solvents is to be obtained. In contrast, decarboxylation step iv) is not necessary when an extract comprising CBGA (acid) or CBDA (acid) free of waxes and reaction solvents is to be prepared.

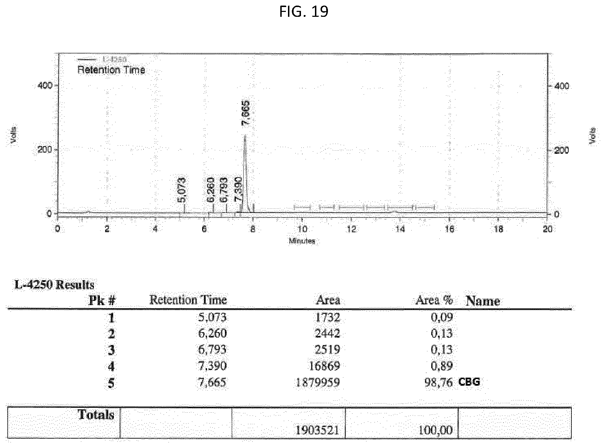

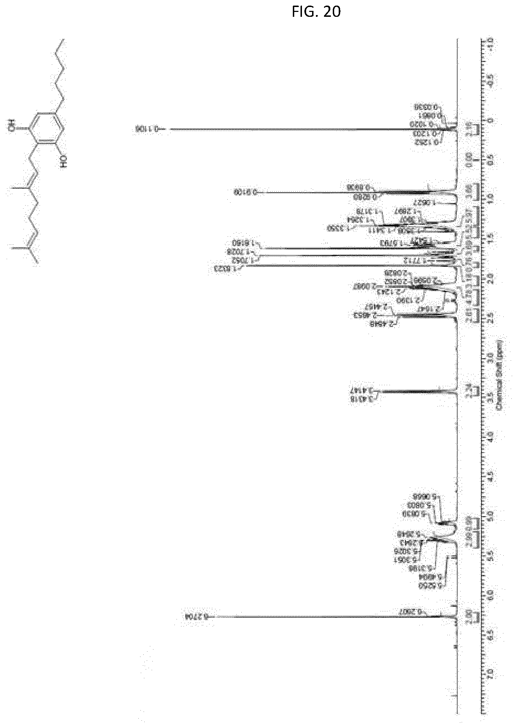

Therefore, decarboxylation step iv) can be omitted and, if desired, the CBGA and/or CBDA can be crystallized to obtain CBGA pure crystal (Examples 12 and 13) and/or CBDA pure crystal. Alternatively, the decarboxylation step can be deferred and carried out on CBGA pure crystal (Fig. 19 and Fig. 20) or CBDA pure crystal.

Thus, the process of the invention may comprise a cannabidiolic acid (CBDA) and/or cannabigerolic acid (CBGA) crystallization step, preferably conducted by concentrating the organic phase obtained from step iii) to oil and adding heptane or hexane to said oil. Optionally, CBDA and/or CBGA pure crystal can then be subjected to decarboxylation to obtain the corresponding neutral cannabidiols. As will be evident from the examples, the process according to this embodiment of the invention was found to be particularly suitable for the preparation of pure crystal CBGA/CBG.

Alternatively, the acid cannabinoids contained in the organic phase obtained from phase iii) can be salified and crystallized. As will become apparent to a technician in the field, the salification of acid cannabinoids can be carried out according to any of the methods known in the art, e.g., by adding to the organic phase obtained from phase iii), possibly concentrated, an organic base, preferably a primary, secondary, tertiary amine with up to 48 carbon atoms such as dicyclohexylamine. Other bases suitable for salt formation are ammonia, alkoxides, hydroxides, carbonates, hydrogen carbonates, carboxylates and other basic salts of first, second and third group elements and of tin, lead and bismuth, and other basic salts of transition elements such as, for example, silver (Ag+). Inorganic salts can also be complexed (e.g., aluminum hydroxide as a silver diamine complex) to increase solubility. Suitable solvents are, for example, alcohols, esters, ethers, ketones, hydrocarbons, halogenated hydrocarbons and nitriles with up to 20 carbon atoms, preferably ethanol.

As an example, crystallization of the ammonium salt of CBDA can be carried out by passing through a salification using dimethylcyclohexylamine in ethanol to obtain the corresponding CBDA salt pure crystal (Example 14). In addition, the ammonium salt of CBDA pure crystal can be decarboxylated to obtain CBD pure crystal (Example 15).

In other words, and to summarize, decarboxylation step iv) is optional and/or can be carried out directly after counter-extraction step iii) with organic solvent or after crystallization of the acid cannabidiols contained in the organic phase obtained from step iii) or again after salification and crystallization of the acid cannabinoids contained in the organic phase obtained from step iii).

The purity and weight yields of the crystalline forms of CBD and CBG are higher if they are obtained through transition to the respective acidic or salified forms as pure crystals, as compared to obtaining them through direct decarboxylation of the acidic forms in an oily state.

In addition, the salified forms allow significantly lower temperatures and decarboxylation times, are more stable than the oily forms, and are more easily stored; important factors from the perspective of industrializing the process.

Preferably according to the present invention, the raw material used is industrial hemp (*Cannabis Sativa* L species; *Sativa* subspecies), but this invention can be applied to any species of the genus *Cannabis.*

The cannabis plant biomass may be fresh, dried or frozen and is preferably shredded before being subjected to extraction according to the process of the present invention.

According to the present invention, the cannabinoids are separated from the waxes, chlorophylls, and any fatty acids that remain in the biomass, and thus the solution can be directly usable for subsequent processing steps.

Besides being simple and inexpensive, the present process avoids a number of the drawbacks of known processes related, for example, to the use of highly flammable solvents or the use of high pressures, and thus is overall safer and easier to implement and scale-up industrially.

The purity of the extracts against the simplicity of the process is also extremely high, allowing the extracts from the process to be used as such without the need for any purification, particularly without resorting to purification by chromatography.

Furthermore, as will be evident from the following examples, the process of the invention is equally applicable to cannabis biomass having a different CBD / CBDA content (Example 2, Example 3, Example 5 and Example 9). Without wishing to be bound by any particular theory, the inventors believe that this is possible because of the step of controlling, and correcting where necessary, the pH by step ii) of the present process.

The following examples are given for illustrative and non-limiting purposes of the present invention.

### EXAMPLES

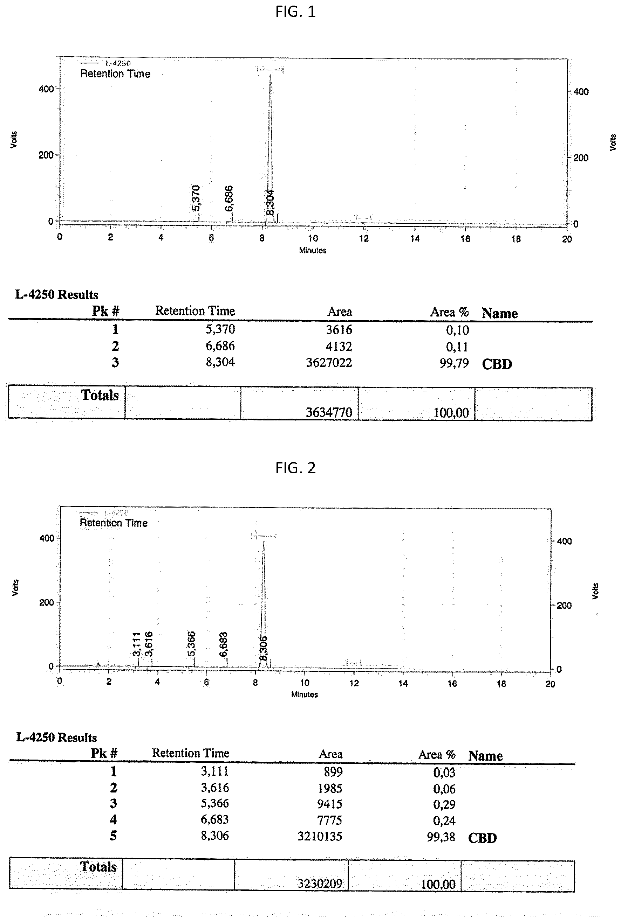

### HPLC working conditions (Fig. 1, 2 and 3):

| | |
|---|---|
| SYSTEM | HPLC Merck Hitachi Shimadzu column |
| Column | Raptor ARC-18 2.7 µm, 150x3.0 mm |
| Mobile phase | Buffer: water + 85% orthophosphoric acid (8.65 g\l) premixed |
| | Phase A: 36% buffer + 64% acetonitrile |
| | Phase B: 18% buffer + 82% acetonitrile |
| Gradient | 0 min 100% A, 16 min 100% A, 17 min 100% B, 20 min 100%A |
| Wavelength | UV 225 nm |
| Flow rate | 1 ml\min |
| Injection volume | 10µL |
| Elution time | 20 minutes |

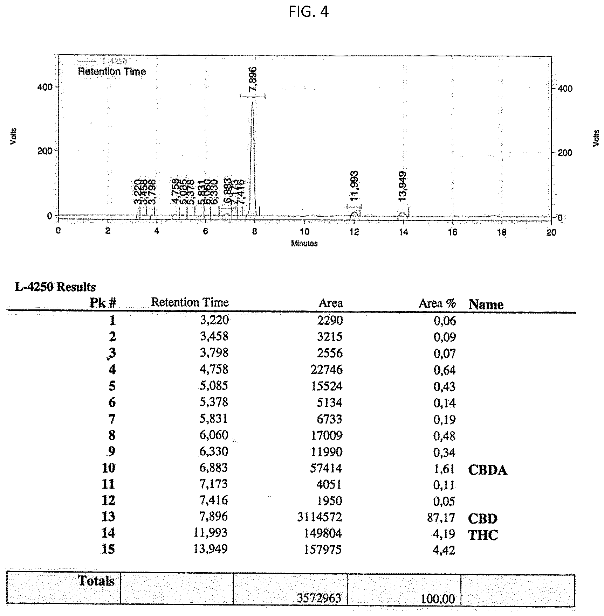

### HPLC working conditions (Fig. 4, 5, 6, 7, 8 and 9):

| | |
|---|---|
| SYSTEM | HPLC Merck Hitachi Shimadzu column |
| Column | Raptor ARC-18 2.7 µm, 150x3.0 mm |
| Mobile phase | Buffer: water + 85% orthophosphoric acid (8.65 g\l) premixed |
| | Phase A: 36% buffer + 64% acetonitrile |
| | Phase B: 18% buffer + 82% acetonitrile |
| Gradient | 0 min 100% A, 16 min 100% B, 17 min 100% A, 20 min 100%A |
| Wavelength | UV 225 nm |
| Flow rate | 1 ml\min |
| Injection volume | 10µL |
| Elution time | 20 minutes |

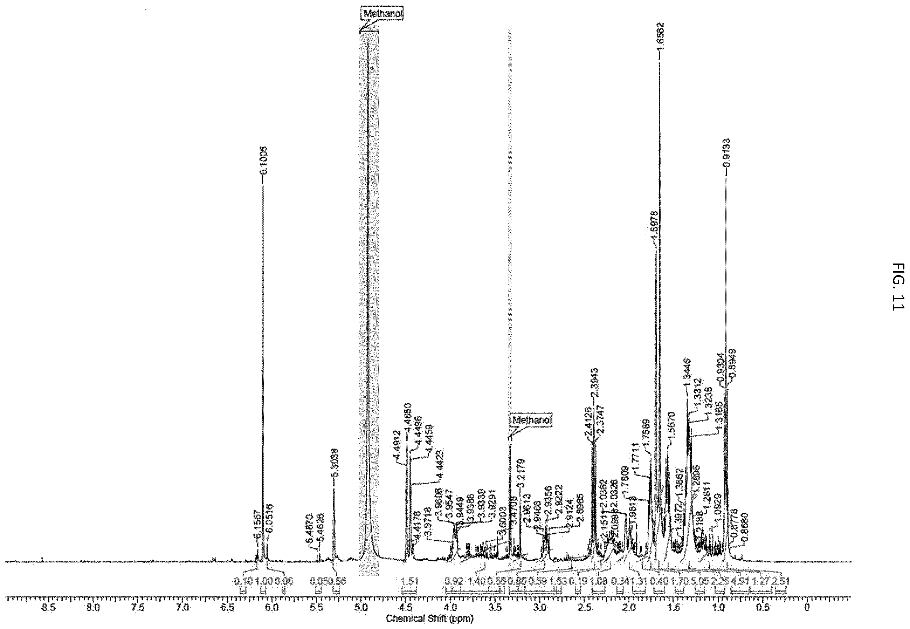

### NMR working conditions (Figs. 11, 12 and 13):

NMR spectra were recorded by dissolving the sample in CD₃OD at a temperature of 25°C using a Bruker 400 instrument, working at 400 MHz for ¹H.

### EXAMPLE 1: Obtaining a wax-free hemp extract by extraction of plant biomass using a mixture of water and ethanol.

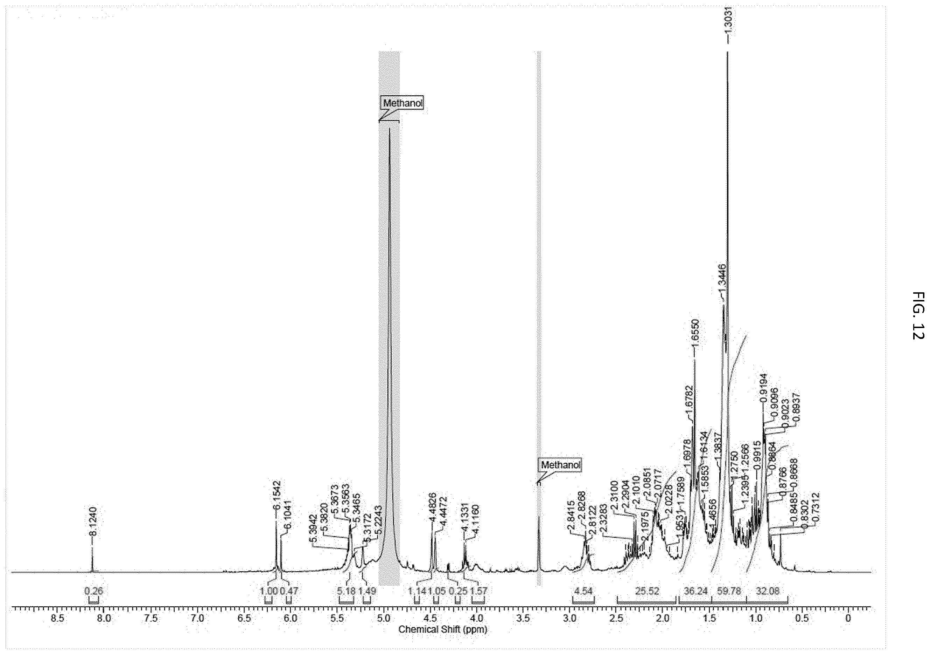

In a 5-liter beaker under stirring, 2022 g of ethanol and 2022 g of water are added then, at room temperature, the 500 g of dried and shredded hemp shredder with a CBDA + CBD content of about 6% is added; it is left stirring for 2 hours without pH correction. After this time, the pH that was at 5.8 was corrected with a 30% KOH solution to pH 7.7; leave in agitation for one hour and filter the biomass, which was washed with a hydroalcoholic solution containing 1011 g ethanol and 1011 g water. The hydroalcoholic solution and washes are pooled and concentrated to reduce the volume to 50%. A small aliquot was concentrated to verify the absence of the waxes already from the crude extract. As can be seen in Fig. 11, corresponding to the proton NMR spectrum of the full spectrum extract, there is no presence of the characteristic aliphatic signals attributable to the presence of waxes in the high field zone with chemical shift between 0-2.5 ppm. In addition, it was possible to isolate the waxes confined in the hemp and perform proton NMR analysis as shown in Fig. 12 (see Example 9 test 2), in which the aliphatic proton signals are evident due to the chemical nature of the waxes, with the very characteristic fingerprints making it easy and unambiguous to distinguish between the signals due to the cannabinoid active ingredients and the waxes.

### EXAMPLE 2: Hydroalcoholic extraction (ethanol and water) from fresh hemp, counter-extraction of the filtrate with heptane, and decarboxylation to obtain a wax-free extract to crystallize pure CBD

In a 5-liter beaker, under stirring, 1800 g of ethanol and 1100 g of water are added then, at room temperature, 934 g (the water content present in fresh hemp is about 75 percent so it is 700 g, this justifies the lower amount of water put in the starting hydroalcoholic solution) of manually shredded fresh hemp with a CBD content of about 10% on the dried substance, the suspension is left stirring for about 3 hours after which the pH is corrected to 8.8 with potash. The biomass is filtered and washed with a hydroalcoholic solution containing 790 g ethanol and 1000 g water. The hydroalcoholic solution and washes are combined and concentrated, yielding 82 g of concentrated product in the form of oil.

The oil is taken up with 79 g ethanol, 100 g water and 124 g heptane, heated to homogenize, the pH is corrected to 5.2 and the phases are separated. The hydroalcoholic phase is removed, the organic phase is concentrated to oil, resulting in 26 g, which is taken up with 52 ml of methanol to check for the absence of waxes. The solution kept at both room temperature and -20°C overnight is clear.

The methanolic solution is again concentrated to oil to proceed to decarboxylation and crystallization of CBD with heptane, yielding 9.2 g of wax-free CBD (Fig. 1).

Counter-extraction with heptane results in a cleaner extract because the more polar products remain in the hydroalcoholic phase, facilitating when necessary the crystallization of CBD.

### EXAMPLE 3: Hydroalcoholic extraction (ethanol and water) from dried hemp having a CBDA/CBD ratio of about 60/40, counter-extraction with heptane and decarboxylation to obtain a wax-free extract to crystallize a pure CBD

In a 3-liter beaker under stirring, 1050 g of ethanol and 1060 g of water are added then, at room temperature, the 300 g of shredded dried hemp with a CBDA + CBD content of about 10% is added; it is left stirring for 4 hours after which the pH is corrected to 8.5/8.7 with soda. The biomass is filtered and washed with a hydroalcoholic solution containing 660 g ethanol and 670 g water. The hydroalcoholic solution and washes are combined and concentrated, yielding 98 g of concentrated product.

The oil is taken up with 83 g heptane isomer mixture, with 57 g ethanol and 60 g water and heated to homogenize it, the pH is corrected to 5.2 and the phases are separated. The hydroalcoholic phase is removed, the organic phase is concentrated to oil, resulting in 45 g, which is taken up with 50 ml of methanol to check for the absence of waxes.

The solution held at both room temperature and -20°C overnight is clear.

The methanolic solution is again concentrated to oil to proceed with decarboxylation and subsequent crystallization of CBD with heptane, resulting in 14 g of pure crystal CBD free of waxes (Fig. 2).

### EXAMPLE 4: Hydroalcoholic extraction (ethanol and water) from dried hemp.

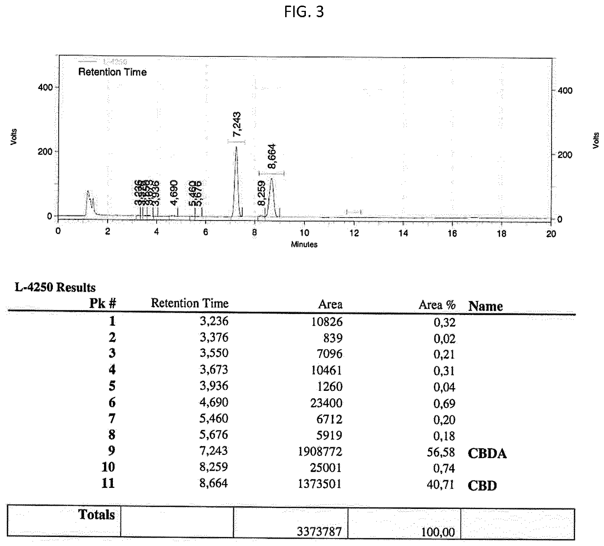

In a 1-liter beaker under stirring, 425 g of ethanol and 425 g of water are added then, at room temperature, 105 g of shredder dried hemp is added, allowed to stir for 30 minutes and then the pH is corrected to 8 with soda ash. The biomass is filtered, which is washed with a hydroalcoholic solution containing 213 g ethanol and 213 g water, the pH is corrected to 5.2 with acetic acid and concentrated by rotavapor to oil, yielding 25 g, 29 g heptane, 20 g ethanol and 21 g water are added to the oil, the phases are separated and the organic phase is concentrated to oil; yielding a wax-free oil whose content is given by the sum of purified CBD and CBDA (Fig. 3).

The oil can be used as such or decarboxylated to obtain a purified and decarboxylated product or used for CBD pure crystal crystallization.

### EXAMPLE 5: Hydroalcoholic extraction from dried hemp having a CBDA/CBD ratio of about 85/15, counter-extraction with heptane and decarboxylation to obtain a wax-free extract to crystallize a pure CBD

In a 3-liter beaker under stirring, 1011 g ethanol and 1011 g water are added then, at room temperature, 250 g of dried hemp shredded with a CBDA + CBD content of about 6 percent is added; it is left stirring for 2 hours without pH correction since the measured value was 6.7. The biomass is filtered and washed with a hydroalcoholic solution containing 506 g ethanol and 506 g water. The hydroalcoholic solution and washes are combined and concentrated, yielding 65 g of product.

The oil is taken up with 69 g of n-heptane, with 48 g of ethanol and 50 g of water being heated to homogenize it, the pH is corrected from the initial 6.7 to 5.2, and the phases are separated. The hydroalcoholic phase is removed, the organic phase is concentrated to oil, resulting in 22 g, which is taken up with 50 ml of methanol to check for the absence of waxes.

The solution kept at both room temperature and -20°C overnight is clear.

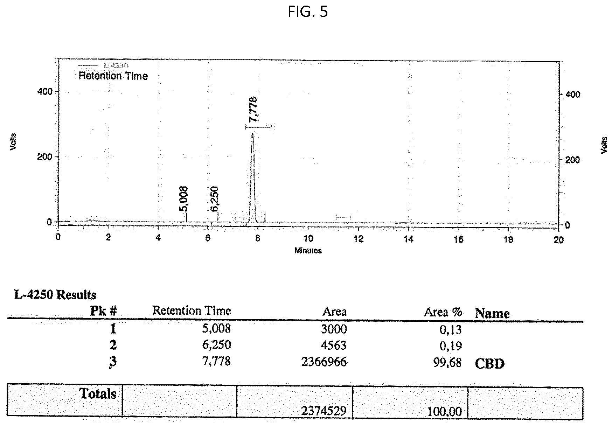

The methanolic solution is again concentrated to oil to proceed to decarboxylation (Fig. 4) and then crystallization of CBD with heptane (Fig. 5), yielding 3.5 g of pure crystal CBD free of waxes.

### EXAMPLE 6: Double hydroalcoholic extraction from dried hemp, counter-extraction of the combined solutions to obtain a wax-free extract and decarboxylation to crystallize a pure CBD

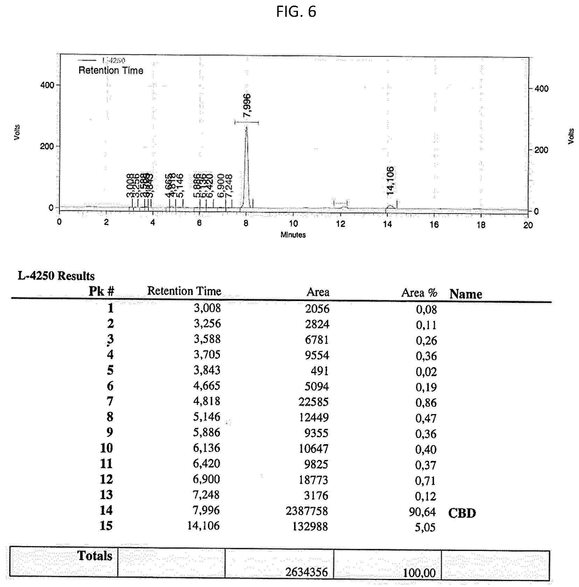

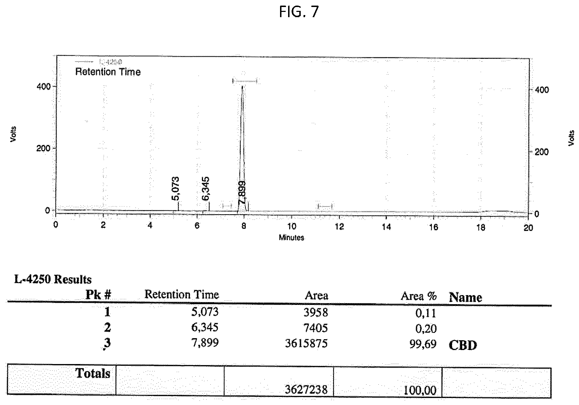

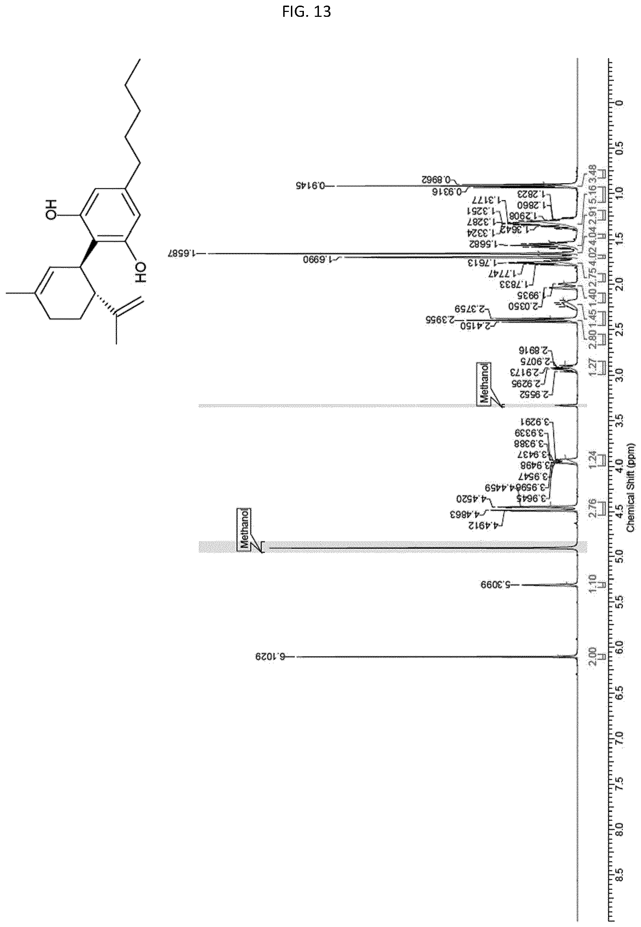

In a 5-liter beaker under stirring, 2022 g of ethanol and 2022 g of water are added then, at room temperature, 500 g of shredded dried hemp with a CBDA + CBD content of about 6% is added; it is left stirring for 30 minutes after which the pH is corrected to 7.8 with soda. After an hour, the biomass is filtered and washed with a hydroalcoholic solution containing 1012 g ethanol and 1012 g water. Another extraction is made in the usual manner and using the same biomass. The combined solutions are concentrated to oil, yielding 257 g; 190 g ethanol and 200 g water are added; the pH from 6.8 is raised to 5 with acetic acid; finally, 276 g heptane is added; the phases are separated, the hydroalcoholic phase is removed, and the organic phase is concentrated to oil, yielding 104 g; it is decarboxylated at 130°C for 3 hours, followed by dilution with 3 volumes of heptane and filtered over 24 g silica. The filtered solution is concentrated, obtaining 90 g of oil (Fig. 6) then from the purified oil it is crystallized from heptane, obtaining 25 g of CBD pure crystal (Fig. 7 and Fig. 13).

### EXAMPLE 7: Extraction with water and acetone from dried hemp to obtain a wax-free extract, counter-extraction with heptane and decarboxylation to crystallize a pure CBD

In a 3-liter beaker under stirring, 1000 g of acetone and 1000 g of water are added then, at room temperature, 250 g of dried shredded hemp is added, left stirring for 1 hour without pH correction after which the pH is corrected from 6.4 to 7.8. The biomass is filtered and washed with a solution containing 500 g acetone and 500 g water. The solution and washes are combined and concentrated, yielding 80 g of product.

Concentration can be advantageous during industrialization to reduce volumes. Next, the ratios of water and ethanol solvents are restored according to the present invention.

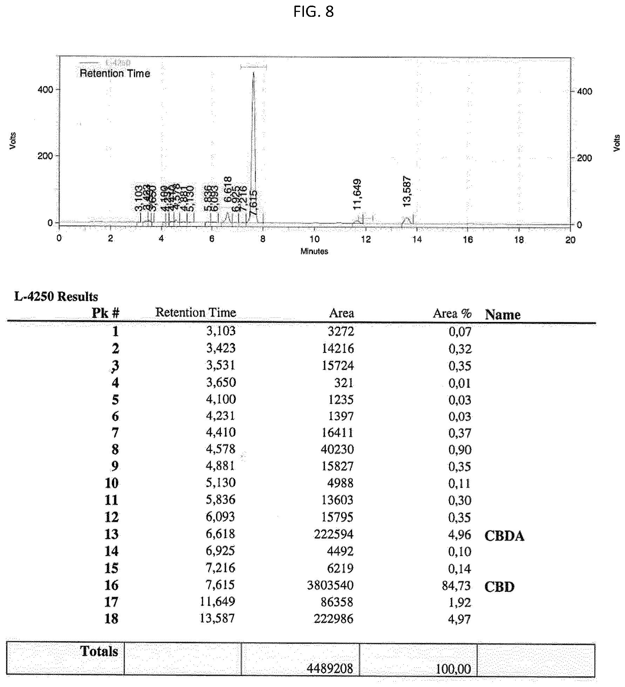

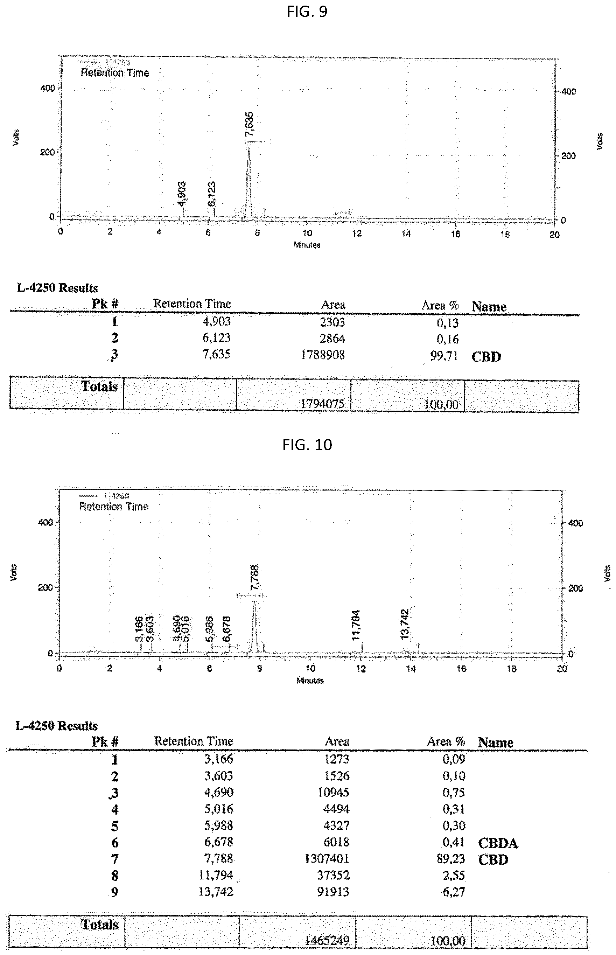

The oil is taken up with 48 g ethanol and 50 g water, the pH is raised to 5.2, 69 g heptane is added, and the phases are separated. The hydroalcoholic phase is removed, the organic phase is concentrated to oil and decarboxylated (Fig. 8); after decarboxylation, it is diluted with 2 volumes of heptane and filtered over 6 g silica. The filtered solution is concentrated then heptane is added for crystallization, resulting in 6.7 g of CBD pure crystal (Fig. 9).

### EXAMPLE 8: Extraction with water and ethanol at a temperature of 50°C from dried hemp to obtain a wax-free extract counter-extraction with heptane and decarboxylation to crystallize a pure CBD

In a 2-liter beaker under stirring, 300 g of ethanol and 600 g of water are added then, at a temperature of 50°C, 105 g of dried shredded hemp is added, left stirring for 30 minutes without pH correction subsequently the pH is corrected from 6.3 to 7.5 with a 30% KOH solution. After 2 hours, the biomass is filtered and washed with a solution containing 300 g ethanol and 600 g water. The solution and washes are combined and concentrated, yielding 28.3 g of product.

The oil is taken up with 40 g ethanol and 42 g water the pH is raised to 4.9 with 85 percent formic acid, then 58 g heptane is added and the phases are separated. The hydroalcoholic phase is removed, the organic phase is concentrated to oil and decarboxylated, yielding 3.9 g of oil, to the latter 2 volumes of methanol are added to test for the absence of waxes, the solution is placed at -20°C overnight, after this period the solution remained clear, so no wax content was evidenced. The methanol is concentrated, returned to syrup and crystallized from heptane, resulting in 1.8 g of pure crystal CBD.

### Example 9: Extraction with hemp at 34 % moisture content and stored at -20°C counter-extraction in heptane, decarboxylation and filtration on silica with subsequent extraction of waxes from spent biomass

### 1. Extraction from biomass

In a 3-liter beaker under stirring 1000 g ethanol and 1000 g water are added, then 338 g frozen hemp with a moisture content of 34% is added under stirring, allowed to stir for 30 minutes without pH correction (pH 5.7). The pH is corrected to 7.9 with a 30% NaOH solution, after 90 minutes the biomass is filtered and washed with a solution containing 500 g ethanol and 500 g water. The solution and washes are combined and concentrated, yielding 74.4 g of product.

The oil is taken up with 47.5 g ethanol and 50 g water, the pH is raised to 5.2 with glacial acetic acid, then 69 g heptane is added and the phases are separated. The hydroalcoholic phase is removed, and the organic phase is concentrated to oil and decarboxylated, yielding 23.5 g of oil. The oil is diluted with 2 volumes of heptane, and the solution is filtered over 6 g of silica previously moistened with heptane for further purification from polar products related to silica. It is returned to syrup and crystallized from heptane, yielding 8.1 g of pure crystal CBD.

### 2. Extraction from spent hemp biomass

The spent hemp was washed with 2 I ethanol and the solution was concentrated to syrup, yielding 14 g of residue. To this was added 120 ml of ethanol, 80 ml of water and 80 ml of heptane, the phases were separated, the hydroalcoholic phase was removed and the organic phase was decolorized with charcoal. The organic solution is concentrated to oil and analyzed for the presence of waxes.

From Fig. 12, it can be appreciated that H¹NMR analysis of the confined waxes in hemp highlights the presence of the aliphatic proton signals due to the chemical nature of the waxes, which exhibit a very characteristic fingerprint that makes it easy and unambiguous to distinguish between the signals due to the cannabinoid active ingredients and the waxes.

### EXAMPLE 10: Material from Example 1 free of waxes and decarboxylated after step ii).

In a 3-liter flask was put all the solution from the extraction of Example 1 that had been partially concentrated to decrease some of its volume, this solution was brought to a temperature of 80 °C for 30 h to carry out decarboxylation directly from the extraction solution. After decarboxylation (Fig. 10), the solution was concentrated to oil, yielding 168 g. The oil was purified by adding 96 g of ethanol and 100 g of water then, at room temperature, the pH was corrected to 4.8 with formic acid; 140 g of heptane was added to the solution and the phases were separated; the organic phase was concentrated to oil, yielding 75 g while the hydroalcoholic phase was removed.

### EXAMPLE 11 (comparative)

### a. Effect of phase ii) of pH correction.

Extraction performed without pH correction with a 50% ethanol solution results in the extraction of 48% of the total amount of CBD in the starting plant material (see, for example Table 1 and Table 3 of U.S. Patent Application US 2018/280459 A1).

In order to be able to assess the cannabinoid content in the starting plant material, this material is analyzed according to the method given in the German Pharmacopoeia (*New Text of the German Pharmacopoeia, Notified in accordance with Directive (EU) 2015*/*1535 of the European Parliament and of the Council of September 9, 2015 laying down a procedure for the provision of information in the field of technical regulations and of rules on Information Society services (OJ L 241, 17. 9.2015, p 1),* the same analytical method is also applied to the spent plant material obtained as a result of extraction conducted following the method of the present invention.

Following the method of the invention, in which the extraction of the cannabinoid active ingredients is carried out in two steps, step i) of extraction without pH correction and step ii) in which the pH of the suspension is corrected, results in a higher extraction of the content of cannabinoids present in the starting plant material, as shown in the Tables below.

**Table 1**

| **Hemp to be extracted** | **CBDA HPLC Area** | **CBD HPLC Area** |
|---|---|---|
| Dried hemp 1 used in Examples 14 and 15 | 6706811 | 1427168 |
| Hemp 2 frozen at 34% moisture content used in Examples 9 and 14 | 3781431 | 289332 |
| **Exhausted hemp** | **CBDA HPLC Area** | **CBD HPLC Area** |
| Hemp 1 spent used in Examples 14 and 15 | 299103 | 114295 |
| Hemp 2 used in Examples 9 and 14 | 143243 | 33077 |

**Table 2**

| **Hemp to be extracted** | **CBGA HPLC area** | **CBG HPLC area** |
|---|---|---|
| Dried hemp 3 with high CBG content used in Examples 12 and 13 | 3017977 | 68556 |

| **Exhausted hemp** | **CBG HPLC Area** | **CBGA HPLC Area** |
|---|---|---|
| Hemp 3 spent used in Examples 12 and 13 | ND | ND |

**Table 3: Percentage of main cannabinoids extracted versus starting.**

| | |
|---|---|
| Hemp 1 | 95% |
| Hemp 2 | 96% |
| Hemp 3 | 100% |

This comparative example demonstrates that step ii) of pH correction to values between 6.5 and 10.5 results in a higher extraction of the major cannabinoids present in the starting plant material compared to procedures in which the extraction does not include a pH correction step, such as the procedure reported in US 2018/280459 A1, which instead result in a partial extraction of the cannabinoid content (48% for total CBD).

In the Examples according to the present invention, while starting from three different types of plant material, as denoted in Table 1 and Table 2, it was possible to obtain extractions whose total CBD or CBG content was found to be >95%.

### b. Comparison with respect to the method described in International Patent Application Publication No. WO 2019/057994 A1.

### Test A and Test B:

850 g of ethanol and 850 g of water are added to a 3-liter beaker under stirring, then 210 g of hemp is added under stirring, allowed to stir for 10 minutes without pH correction (pH 5.7). The pH is corrected to 8.5 with a 30 percent NaOH solution, after 180 minutes the biomass is filtered and washed with a solution containing 425 g ethanol and 425 g water. The solution and washes are combined, resulting in 2696 g of extract in hydroalcoholic solution that is divided into two equal portions of 1348 g.

**Test A:** 1348 g of extract in hydroalcoholic solution is extracted according to the present invention, that is, 700 ml of water is added, the pH is raised to 4.5 with formic acid, then 300 ml of heptane is added and the phases are separated. The hydroalcoholic phase is removed, the organic phase concentrated to oil, yielding 9.4 g of oil.

**Test B:** 1348 g of extract in hydroalcoholic solution is concentrated under vacuum in rotavapor until the ethanol is completely eliminated, 175 ml of ethyl ether is added to the remaining aqueous solution, the pH is raised to 6.07 with a 5% citric acid solution, and the phases are separated.

The organic phase is washed twice with 70 ml deionized water, the phases are separated and the organic phase is concentrated, obtaining 10.9 g of oil.

The oil is taken up with 20 ml of methanol and brought to -20°C, showing the absence of the waxes through no precipitate formation.

**Test C:** in a one-liter beaker under stirring, 30 g of hemp and 200 ml of saturated aqueous solution of K2CO3, whose pH at 22°C turns out to be 14, are added, 200 ml of ethanol is added and left stirring for one hour, the pH of the resulting hydroalcoholic solution at 22°C turns out to be 14. Then, the hydroalcoholic solution is filtered to separate the plant biomass, the aqueous and alcoholic phases turn out to be immiscible and are separated.

The aqueous phase is removed, while the ethanol phase is concentrated under vacuum in rotavapor, obtaining 5.2 g of red-colored oil. The oil is imaged with 10 ml of methanol and brought to -20°C, showing the presence of the waxes through the formation of a precipitate.

**Test D:** n a one-liter beaker under stirring, 30 g of hemp and 200 ml of saturated aqueous solution of K2CO3, whose pH at 22°C is found to be 14, are added and left stirring for one hour. Next, the aqueous solution is filtered to separate the plant biomass, and then removed, while 200 ml of 90% ethanol is added to the plant biomass under agitation, left to agitate for an additional 30 minutes and filtered, and the plant biomass is washed with 30 ml of 90% ethanol. The ethanolic phases are combined and concentrated under vacuum in rotavapor obtaining 12.5 g of red-colored oil.

To the 12.5 g of oil, 50 ml of ethyl ether is added, the pH is raised to 6.0 with a 5 percent citric acid solution, and the phases are separated. The organic phase is washed twice with 20 ml deionized water, the phases are separated and the organic phase is concentrated, obtaining 2.7 g of oil.

The oil is taken up with 5.5 ml of methanol and brought to -20°C, showing the presence of the waxes through the formation of a precipitate.

**Table 4 comparing the % areas in HPLC of the oils obtained in the four tests**

| | Start | **Test A*** | **Test B**** | **Test C***** | **Test D****** |
|---|---|---|---|---|---|
| CBDA (% area) | 70,73 | 79,50 | 71,39 | 61,89 | 56,47 |
| CBD (% area) | 15,05 | 12,91 | 19,39 | 27,28 | 29,34 |
| Total | 85,78 | 92,41 | 90,74 | 89,17 | 85,81 |

| | | | | | |
|---|---|---|---|---|---|
| *Test A: according to the extraction process of the present invention. **Test B: steps i) and ii) according to the extraction process of the present invention but step iii) different i.e. extraction with ethyl ether as provided by the method described in WO 2019/057994 A1 instead of with solvent immiscible with the hydroalcoholic mixture used in steps i) and ii). Under these conditions, an oil is obtained whose analytical profile contains more impurities in the absence of waxes. The test for evaluating the presence of waxes in the extract has been described in this description. ***Test C: According to the extraction process described in WO 2019/057994 A1, which involves incubating the starting plant material in an aqueous medium including a saturated solution of K₂CO₃ in a hydroalcoholic mixture and then at pH 14. Under these conditions, phenate formation occurs with red color (characteristic) of the resulting concentrated oil in which waxes are evident after keeping the oil in methanolic solution at -20°C. ****Test D: According to the extraction process described in Example 1 of WO 2019/057994 A1 which involves incubating the starting plant material in an aqueous medium comprising a saturated solution of K₂CO₃, removal of the aqueous phase by filtration from the hemp and addition on the hemp of 90 percent ethanol while maintaining under stirring for an additional 30 minutes. Under these conditions, the oil-concentrated extract is extracted with ethyl ether through pH correction to 6 with a 5 percent citric acid solution, following phase separation in the resulting concentrated oil, the presence of waxes is revealed after keeping the oil in methanolic solution at -20°C. | | | | | |

As can be seen from the experimental data in Table 4, the extraction process of the present invention allows for extracts with a higher degree of purity than the processes already known in the state of the art. In addition, an extract with a higher total CBDA/CBD ratio is obtained, which may be important, for example, if the crystallization of the acid form, in acid or salt form, is desired.

Confirming the advantages of the extraction process according to the present invention, it can be noted that in Tests A and B, which follow the extraction method of the invention, the presence of waxes was not found in the oil obtained. Differently in Tests C and D, which were carried out according to the methods reported in WO 2019/057994 A1, the process in both cases resulted in obtaining oil in which the presence of the waxes was evidenced through the experimental test.

### EXAMPLE 12: Obtaining a high CBG/CBGA wax-free hemp extract by hydroalcoholic extraction of plant biomass and counter-extraction in heptane to obtain CBGA pure crystal.

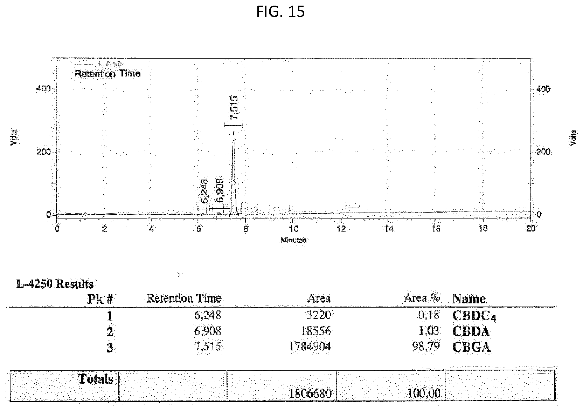

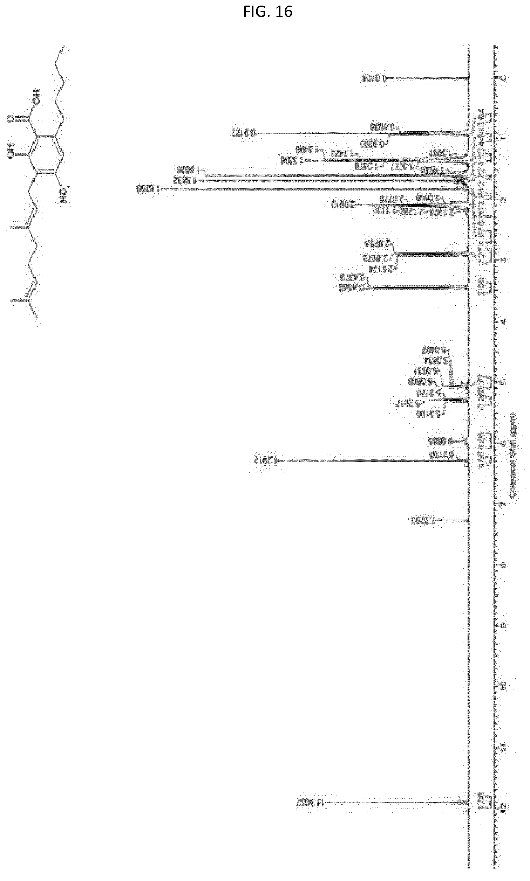

In a 2-liter beaker under stirring, 750 g of ethanol and 750 g of water are added then, at room temperature, 105 g of dried hemp plant material with a CBGA + CBG content of about 5% is added. It is left stirring for 3 hours without pH correction, after which the pH - which was 5.5 - is corrected with a 30% NaOH solution and raised to pH 9.0. It is left stirring for 3 hours and the biomass is filtered and washed with a hydroalcoholic solution containing 250 g ethanol and 250 g water twice. The hydroalcoholic solution and washes are combined 300 ml of heptane is added, the pH is corrected to 4.5 with formic acid and the phases are separated. The hydroalcoholic phase is counter extracted with another 300 ml of heptane. The two organic solutions are combined and concentrated to oil, 18 g is obtained and 90 ml of heptane is added to it, the product begins to crystallize, the suspension is placed at 4°C overnight, and the solid is filtered and washed with 12 ml of cold heptane. 4.8 g of pure crystal of CBGA free of waxes is obtained. (Fig. 15 and Fig.16)

### EXAMPLE 13: Obtaining a high CBG/CBGA wax-free hemp extract by hydroalcoholic extraction of 2 kg of plant biomass to obtain CBGA pure crystal.

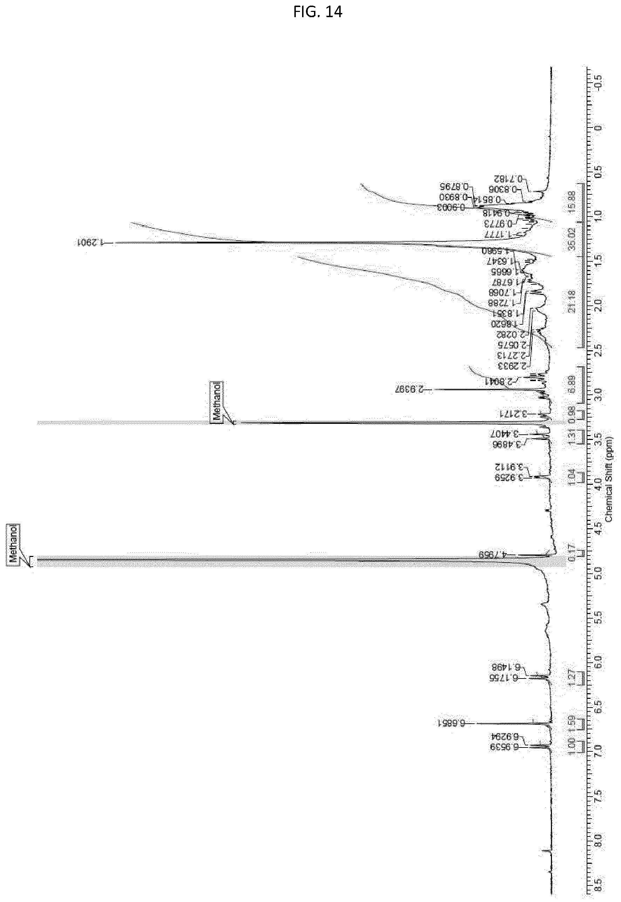

In a 50-liter reactor under stirring, 15.3 kg of ethanol and 15.3 kg of water are added then, at room temperature, 2 kg of dried hemp plant material with the same characteristics as in Example 1 is added. It is left stirring for 15 minutes without pH correction. After that, the pH - which was below 6.5 - is corrected with 815 ml of a 30% NaOH solution to pH 8.0. It is left stirring for 1 hour and then allowed to stand overnight; the biomass is filtered, which is fortified with a hydroalcoholic solution containing 7.5 kg ethanol and 7.5 kg water and left stirring for 1 hour; the biomass is again fortified with a hydroalcoholic solution containing 7.5 kg ethanol and 7.5 kg water, left stirring for 1 hour; and the biomass is filtered. This is dried (LOD 8.1%) and after extraction with absolute ethanol, the resulting solution concentrated and analyzed by NMR (Fig.14) for the presence of waxes. The hydroalcoholic solution and washes are pooled and weighed, resulting in 56.5 kg.

1/40 of the hydroalcoholic solution (1412 g) is taken, to this is added 706 g of water and 150 ml of heptane, left to stir correct the pH to 4.5 with formic acid, separate the phases, the hydroalcoholic phase is removed and the organic phase concentrated to oil, 5.1 g is obtained and to this 20 ml of heptane is added, the product begins to crystallize, the suspension is placed at 4°C overnight, the solid is filtered and washed with 10 ml of cold heptane. 2.8 g of pure crystal of CBGA free of waxes is obtained.

It is highlighted that the absence of the waxes in the hydroalcoholic extracts is confirmed by further addition of water, a solvent in which they have lower solubility, leading to precipitation phenomena. The lack of precipitation is a confirmation that they remain confined in the biomass. In addition, NMRs performed on the obtained products confirmed the absence of the waxes, which in the case of their presence would have been found in the apolar solvents used for counter-extraction of the aqueous phases, organic solvents in which the waxes have greater affinity.

### EXAMPLE 14: Obtaining a high CBDA hemp extract free of waxes by hydroalcoholic extraction of plant biomass with a water content of 34% to obtain CBDA salt of pure crystal dimethylcyclohexylammonium.

In a 3-liter beaker under stirring, 690 g of ethanol and 635 g of water are added then, at room temperature, 234 g of frozen hemp plant material with 34 percent moisture is added. It is left stirring for 30 minutes without pH correction, after which since the pH was below 6.5 it was corrected with a 30% NaOH solution and brought to pH 8.5. The biomass was left stirring for 2 hours, the biomass was filtered and added with a hydroalcoholic solution containing 200 g ethanol and 200 g water and left stirring for 1 hour; the biomass was filtered and again added with a hydroalcoholic solution containing 200 g ethanol and 200 g water, left stirring for 1 hour and the biomass was filtered.

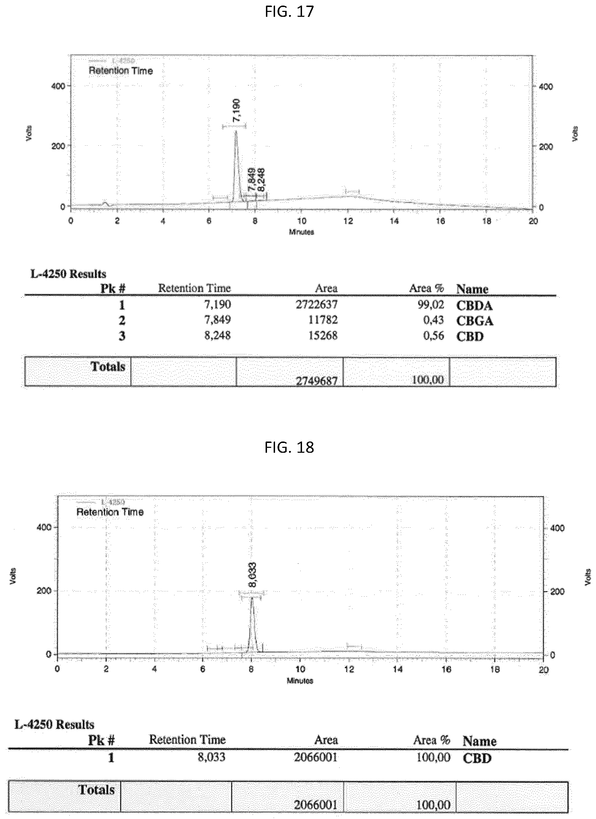

To all the hydroalcoholic solution (1750 g), 875 g water and 200 ml heptane are added, left stirring correct the pH to 4.5 with formic acid, separate the phases, the hydroalcoholic phase is extracted again with 150 ml heptane and the two organic phases combined and concentrated to oil. 14.9 g of oil is obtained and 45 ml of ethanol and 4.2 g of dimethylcyclohexylamine are added to this, the product begins to crystallize, the suspension is placed at 4°C overnight, the solid is filtered and washed with 20 ml of cold heptane. This yields 12.5 g of pure crystal of CBDA dimethylcyclohexylammonium salt free of waxes. (Fig. 17).

### EXAMPLE 15: Decarboxylation of CBDA dimethylcyclohexylammonium salt (DMCEA) pure crystal to prepare CBD pure crystal.

10g of the crystalline solid of Example 15 was taken to which 50g of ethanol was added, the suspension was put under stirring at the solvent reflux temperature (about 80°C), after 4 hours, a red-colored solution is obtained which is analyzed by HPLC confirming total decarboxylation. Next, the solvent is concentrated and 25 g of water, 12.5 g of ethanol and 50 ml of heptane are added by acidifying with acetic acid, stirring and separating the phases, the hydroalcoholic phase is removed while the heptane phase is concentrated to a weight of 8.1g to which 6 ml of heptane is added, resulting in crystallization of CBD, which is filtered and washed with cold heptane obtaining 5.7 g of pure crystal CBD. (Fig. 18).

### EXAMPLE 16: Decarboxylation of pure crystal CBGA to prepare pure crystal CBG.

4.8 g of pure crystal CBGA from Example 12 was taken to which 40 g ethanol and 40 g water were added, the solution was stirred at the solvent reflux temperature (about 80°C), after 5 hours there is complete decarboxylation. Then, the solution is cooled and 50 ml of heptane is added, stirred and the phases are separated, the hydroalcoholic phase is removed while the heptane phase is concentrated to a weight of 2.5 g to which 10 ml of heptane is added, resulting in the crystallization of CBG, which is filtered and washed with cold heptane obtaining 1.9 g of pure crystal CBG. (Fig. 19 and Fig. 20).

The present invention has been described so far with reference to a preferred embodiment. It is to be understood that there may be other embodiments pertaining to the same inventive core, as defined by the scope of protection of the claims reported below.

## Claims

1. A process for producing a wax-free cannabis extract, said process comprising the following steps:
i) suspending a cannabis plant biomass with a mixture of water and water-miscible solvent such that in the suspension the weight ratio of total water to water-miscible solvent is between 3:1 and 1:1 and keeping the suspension under agitation for at least 10 minutes, at a temperature not exceeding 50°C, without correcting the pH;
ii) correct the pH of the suspension obtained from step i) to pH between 6.5 and 10.5, if necessary, and filter away the biomass;
iii) extract the filtrate by addition of an organic solvent immiscible with said mixture, acidify to pH between 5.5 and 4.0, and recover the organic phase.

2. The process according to claim 1, wherein said solvent miscible with water is chosen from alcohols, acetone, acetonitrile or mixtures thereof, preferably wherein said alcohols are chosen from the group consisting of C1-C4 alcohols, more preferably ethanol or methanol.

3. The process according to claim 1 or 2, wherein the temperature is between 0°C and 50°C, preferably between 5°C and 45°C, more preferably between 10°C and 25°C.

4. The process according to any of claims 1 to 3, wherein the suspension is kept under agitation for at least 30 minutes.

5. The process according to any one of claims 1 to 4, wherein said organic solvent is a C5-C8 hydrocarbon, preferably n-heptane or a mixture of its isomers and/or hexane or a mixture of its isomers.

6. The process according to any one of claims 1 to 5, further comprising a step iv) of decarboxylating the filtrate recovered from step ii) or the organic phase recovered from step iii) by heating the material to a temperature between the boiling temperature of the solvent and 180°C.

7. The process according to claim 6 further comprising a step v) of crystallizing cannabinoids, particularly cannabidiol (CBD) and/or cannabigerol (CBG), preferably by adding heptane or hexane to the product obtained from the decarboxylation step **iv).**

8. The process according to any one of claims 1 to 5, further comprising a crystallization step of cannabidiolic acid (CBDA) and/or cannabigerolic acid (CBGA), preferably by concentrating to oil the organic phase obtained from step iii) and adding to said oil heptane or hexane.

9. The process according to any one of claims 1 to 5, further comprising a step of salifying and crystallizing cannabidiolic acid (CBDA) and/or cannabigerolic acid (CBGA) by the addition to the organic phase obtained from step iii), optionally concentrated, of an organic base, preferably dimethylcyclohexylamine, in a suitable solvent.

10. The process according to claim 9 wherein said solvent is selected from alcohols, esters, ethers, ketones, hydrocarbons, halogenated hydrocarbons and nitriles with up to 20 carbon atoms, and is preferably ethanol.

11. The process according to claim 9 or 10 further comprising a step of decarboxylating said salt by heating the material to a temperature between the boiling temperature of the solvent and 180°C to give cannabidiol (CBD) and/or cannabigerol (CBG), and optionally a step of crystallizing CBD and/or CBG thus obtained from heptane or hexane.

12. The process according to any of the preceding claims, wherein said step i) is carried out on fresh, dried or frozen cannabis plant biomass, optionally shredded.

13. The process according to any one of the preceding claims, wherein said cannabis plant biomass is biomass of *Cannabis sativa* L.

## Patentansprüche

1. Verfahren zum Herstellen eines wachsfreien Cannabisextraktes, wobei das Verfahren die folgenden Schritte umfasst:
i) Suspendieren der Biomasse einer Cannabispflanze mit einer Mischung aus Wasser und einem wassermischbaren Lösungsmittel, sodass in der Suspension das Gewichtsverhältnis von Gesamtwasser zu wassermischbarem Lösungsmittel zwischen 3:1 und 1:1 liegt, und Halten der Suspension unter Rühren für mindestens 10 Minuten bei einer Temperatur von nicht mehr als 50 °C, ohne den pH-Wert zu korrigieren;
ii) Korrigieren des pH-Werts der aus Schritt i) erhaltenen Suspension bei Bedarf auf einen pH-Wert zwischen 6,5 und 10,5 und Filtern der Biomasse;
iii) Extrahieren des Filtrats durch Zugabe eines mit der Mischung nicht mischbaren organischen Lösungsmittels, Ansäuern auf einen pH-Wert zwischen 5,5 und 4,0 und Gewinnen der organischen Phase.

2. Verfahren nach Anspruch 1, wobei das mit Wasser mischbare Lösungsmittel aus Alkoholen, Aceton, Acetonitril oder Mischungen davon ausgewählt wird, wobei die Alkohole vorzugsweise aus der Gruppe ausgewählt werden, die aus C1-C4-Alkoholen besteht, bevorzugter Ethanol oder Methanol.

3. Verfahren nach Anspruch 1 oder 2, wobei die Temperatur zwischen 0 °C und 50 °C, vorzugsweise zwischen 5 °C und 45 °C, besonders bevorzugt zwischen 10 °C und 25 °C liegt.

4. Verfahren nach einem der Ansprüche 1 bis 3, wobei die Suspension mindestens 30 Minuten lang unter Rühren gehalten wird.

5. Verfahren nach einem der Ansprüche 1 bis 4, wobei das organische Lösungsmittel ein C5-C8-Kohlenwasserstoff, vorzugsweise n-Heptan oder eine Mischung seiner Isomere und/oder Hexan oder eine Mischung seiner Isomere ist.

6. Verfahren nach einem der Ansprüche 1 bis 5, ferner umfassend einen Schritt iv) des Decarboxylierens des aus Schritt ii) gewonnenen Filtrats oder der aus Schritt iii) gewonnenen organischen Phase durch Erhitzen des Materials auf eine Temperatur zwischen der Siedetemperatur des Lösungsmittels und 180 °C.

7. Verfahren nach Anspruch 6, ferner umfassend einen Schritt v) des Kristallisierens von Cannabinoiden, insbesondere Cannabidiol (CBD) und/oder Cannabigerol (CBG), vorzugsweise durch Hinzufügen von Heptan oder Hexan zu dem aus dem Decarboxylierungsschritt iv) erhaltenen Produkt.

8. Verfahren nach einem der Ansprüche 1 bis 5, ferner umfassend einen Kristallisationsschritt von Cannabidiolsäure (CBDA) und/oder Cannabigerolsäure (CBGA), vorzugsweise durch Konzentrieren der aus Schritt iii) erhaltenen organischen Phase zu einem Öl und Hinzufügen von Heptan oder Hexan zu diesem Öl.

9. Verfahren nach einem der Ansprüche 1 bis 5, ferner umfassend einen Schritt der Salzbildung und Kristallisation von Cannabidiolsäure (CBDA) und/oder Cannabigerolsäure (CBGA) durch Hinzufügen einer organischen Base, vorzugsweise Dimethylcyclohexylamin, in einem geeigneten Lösungsmittel zu der aus Schritt iii), wahlweise konzentriert, erhaltenen organischen Phase.

10. Verfahren nach Anspruch 9, wobei das Lösungsmittel aus Alkoholen, Estern, Ethern, Ketonen, Kohlenwasserstoffen, halogenierten Kohlenwasserstoffen und Nitrilen mit bis zu 20 Kohlenstoffatomen ausgewählt wird und vorzugsweise Ethanol ist.

11. Verfahren nach Anspruch 9 oder 10, ferner umfassend einen Schritt der Decarboxylierung des Salzes durch Erhitzen des Materials auf eine Temperatur zwischen der Siedetemperatur des Lösungsmittels und 180 °C, um Cannabidiol (CBD) und/oder Cannabigerol (CBG) zu erhalten, und wahlweise einen Schritt der Kristallisation des so aus Heptan oder Hexan erhaltenen CBD und/oder CBG.

12. Verfahren nach einem der vorstehenden Ansprüche, wobei Schritt i) an frischer, getrockneter oder gefrorener, wahlweise zerkleinerter Biomasse der Cannabispflanze durchgeführt wird.

13. Verfahren nach einem der vorstehenden Ansprüche, wobei die Biomasse der Cannabispflanze Biomasse von Cannabis sativa L. ist.

## Revendications

1. Procédé de production d'un extrait de cannabis sans cire, ledit procédé comprenant les étapes suivantes :
i) la mise en suspension d'une biomasse végétale de cannabis avec un mélange d'eau et de solvant miscible à l'eau de telle sorte que, dans la suspension, le rapport en poids entre l'eau totale et le solvant miscible à l'eau est compris entre 3:1 et 1:1 et le maintien de la suspension sous agitation pendant au moins 10 minutes, à une température ne dépassant pas 50 °C, sans corriger le pH ;
ii) la correction du pH de la suspension obtenue à l'étape i) à un pH compris entre 6,5 et 10,5, si nécessaire, et le retrait par filtration de la biomasse ;
iii) l'extraction du filtrat par addition d'un solvant organique non miscible avec ledit mélange, l'acidification à un pH compris entre 5,5 et 4,0 et la récupération de la phase organique.

2. Procédé selon la revendication 1, dans lequel ledit solvant miscible à l'eau est choisi parmi alcools, acétone, acétonitrile ou mélanges de ceux-ci, de préférence dans lequel lesdits alcools sont choisis dans le groupe constitué d'alcools en C1-C4, plus préférablement l'éthanol ou le méthanol.

3. Procédé selon la revendication 1 ou 2, dans lequel la température est comprise entre 0 °C et 50 °C, de préférence entre 5 °C et 45 °C, plus préférablement entre 10 °C et 25 °C.

4. Procédé selon l'une quelconque des revendications 1 à 3, dans lequel la suspension est maintenue sous agitation pendant au moins 30 minutes.

5. Procédé selon l'une quelconque des revendications 1 à 4, dans lequel ledit solvant organique est un hydrocarbure en C5-C8, de préférence le n-heptane ou un mélange de ses isomères et/ou l'hexane ou un mélange de ses isomères.

6. Procédé selon l'une quelconque des revendications 1 à 5, comprenant en outre une étape iv) de décarboxylation du filtrat récupéré à l'étape ii) ou de la phase organique récupérée à l'étape iii) en chauffant le matériau à une température comprise entre la température d'ébullition du solvant et 180 °C.

7. Procédé selon la revendication 6 comprenant en outre une étape v) de cristallisation de cannabinoïdes, en particulier le cannabidiol (CBD) et/ou le cannabigérol (CBG), de préférence en ajoutant de l'heptane ou de l'hexane au produit obtenu à l'étape de décarboxylation iv).

8. Procédé selon l'une quelconque des revendications 1 à 5, comprenant en outre une étape de cristallisation de l'acide cannabidiolique (CBDA) et/ou de l'acide cannabigérolique (CBGA), de préférence en concentrant en huile la phase organique obtenue à l'étape iii) et en ajoutant à ladite huile de l'heptane ou de l'hexane.

9. Procédé selon l'une quelconque des revendications 1 à 5, comprenant en outre une étape de salification et de cristallisation de l'acide cannabidiolique (CBDA) et/ou de l'acide cannabigérolique (CBGA) par l'addition à la phase organique obtenue à l'étape iii), facultativement concentrée, d'une base organique, de préférence la diméthylcyclohexylamine, dans un solvant approprié.

10. Procédé selon la revendication 9, dans lequel ledit solvant est choisi parmi alcools, esters, éthers, cétones, hydrocarbures, hydrocarbures halogénés et nitriles ayant jusqu'à 20 atomes de carbone, et est de préférence l'éthanol.

11. Procédé selon la revendication 9 ou 10 comprenant en outre une étape de décarboxylation dudit sel en chauffant le matériau à une température comprise entre la température d'ébullition du solvant et 180 °C pour donner du cannabidiol (CBD) et/ou du cannabigérol (CBG), et facultativement une étape de cristallisation du CBD et/ou du CBG ainsi obtenu à partir d'heptane ou d'hexane.

12. Procédé selon l'une quelconque des revendications précédentes, dans lequel ladite étape i) est réalisée sur de la biomasse végétale de cannabis fraîche, séchée ou congelée, facultativement déchiquetée.

13. Procédé selon l'une quelconque des revendications précédentes, dans lequel ladite biomasse végétale de cannabis est une biomasse de Cannabis sativa L.
